# EUROPEAN PATENT SPECIFICATION

(11) **EP 2 125 690 B1**
(45) Date of publication and mention of the grant of the patent: **09.01.2013**
(21) Application number: 07851791.9
(22) Date of filing: 27.12.2007
(51) Int. Cl.: C07C 51/42

(54) **METHOD AND APPARATUS FOR THE PURIFICATION OF HIGH-PURITY 2,6-NAPHTHALENE DICARBOXYLIC ACID**
VERFAHREN UND APPARAT ZUR AUFREINIGUNG HOCHREINER 2,6-NAPHTHALINDICARBONSÄURE
PROCÉDÉ ET APPAREIL DE PURIFICATION D'ACIDE DE 2,6-NAPHTALÈNEDICARBOXYLIQUE DE GRANDE PURETÉ

(30) Priority: 29.12.2006 KR 20060138701
(43) Date of publication of application: 02.12.2009
(73) Proprietor: Hyosung Corporation, Seoul 121-020 (KR)
(72) Inventor: CHEN, Yang Ho, Mungyeong-si Gyeongsangbuk-do 745-821 (KR); CHOI, Young Gyo, Yongin-si Gyeonggi-do 448-527 (KR)
(74) Representative: Irniger, Ernst
(86) International application number: PCT/KR2007/006864
(87) International publication number: WO 2008/082145

(56) References cited:
- GB-A- 2 187 744
- JP-A- 09 104 654
- JP-A- 09 151 161
- KR-A- 20050 078 684
- KR-A- 20060 078 287

## Description

### Technical Field

The present invention relates to a method and an apparatus for purifying 2,6-naphthalene dicarboxylic acid (NDA) that is present in a solid state in a solution, and more specifically to a method and an apparatus for purifying high-purity 2,6-naphthalene dicarboxylic acid that use sintered metal membrane filtration units to efficiently separate and purify 2,6-naphthalene dicarboxylic acid separated after hydrogenation and crystallization in a continuous process.

**Background Art**

2,6-Naphthalene dicarboxylic acid (NDA) is a useful monomer for the production of polymers, mainly polyethylene naphthalate (PEN). Polyethylene naphthalate is produced by reacting ethylene glycol with 2,6-naphthalene dicarboxylic acid or its dialkyl ester. Polyethylene naphthalate has advantageous characteristics in terms of mechanical properties, heat resistance and gas barrier properties as compared to polyethylene terephthalate (PET). Based on these advantages, polyethylene naphthalate finds important applications in various commercial fields, such as films for magnetic tapes, packages, tire cords and engineering plastics (e.g., liquid crystal polymers).

2,6-Naphthalene dicarboxylic acid is typically produced by oxidation of 2,6-dimethylnaphthalene ('2,6-DMN') in the presence of a heavy metal catalyst. However, the oxidation product, 2,6-naphthalene dicarboxylic acid, inevitably contains large amounts of various impurities. Examples of such impurities include: catalytic metals, such as cobalt and manganese; intermediate oxidation products, such as formylnaphthoic acid ('FNA') and methylnaphthoic acid ('MNA') degradation products, such as trimellitic acid ('TMLA'); brominated products, such as bromonaphthalenedicarboxylic acid ('Br-NDA'); and compounds, such as naphthoic acid ('NA'), derived from impurities contained in the raw material ( i.e. 2,6-DMN).

Direct polymerization of 2,6-naphthalene dicarboxylic acid and ethylene glycol causes serious quality degradation {e.g., poor heat resistance, low softening point and coloration) of the polymerization product (PEN). Thus, 2,6-naphthalenedicarboxylic acid with a purity as high as 99.9% by weight is required to obtain high-quality PEN.

Various purification methods have been proposed to remove impurities present in 2,6-naphthalene dicarboxylic acid. The removal of impurities present in 2,6-naphthalenedicarboxylic acid is essential for the purification of high-purity 2,6-naphthalene dicarboxylic acid. Particularly, according to purification methods suggested in U.S. Patent Nos. 5,256,817 and 6,255,525, a solution of an impure naphthalene dicarboxylic acid in a solvent is subjected to hydrogenation to remove impurities from the impure naphthalene dicarboxylic acid.

Specifically, a crude NDA is dissolved in a solvent, such as acetic acid or an aqueous acetic acid solution (US. Pat. No. 5,256,817) or water (US. Pat. No. 6,255,525) at around 300°C, followed by hydrogenation to remove impurities or convert the impurities to removable forms. A reaction product containing NDA dissolved after the hydrogenation is transferred to crystallizers and cooled to separate pure NDA crystals.GB 2187744 A discloses a process for producing a highly pure produce of 2,6-naphthalenedicarboxylic acid in a large quantity at a moderate price by oxidizing 2,6-diisopropylnaphthalene while using a water-soluble salt of cobalt, a water-soluble salt of manganese or a mixture thereof, a water-soluble salt of cerium and a bromine compound as the catalyst of the oxidation.

Meanwhile, according to prior art purification methods, filtration and rinsing operations after hydrogenation in a batch process require troublesome manual working and the limited treatment capacity results in low yield per unit time.

**Disclosure of Invention**

### Technical Problem

The present invention has been made in an effort to solve the problems of the prior art, and it is one object of the present invention to provide a method and an apparatus for purifying 2,6-naphthalene dicarboxylic acid that use sintered metal membrane filters as high-pressure rinsing equipment to efficiently perform filtration and rinsing after hydrogenation and crystallization in a successive method.

It is another object of the present invention to provide a method and an apparatus for purifying 2,6-naphthalene dicarboxylic acid that allow filtration and rinsing operations after hydrogenation and crystallization to be carried out in a continuous process.

**Technical Solution**

In accordance with one aspect of the present invention for accomplishing the above objects, there is provided a method for separating and purifying 2,6-naphthalene dicarboxylic acid, the method comprising: separating a first mother liquor from 2,6-naphthalene dicarboxylic acid crystals obtained after crystallization using sintered metal membrane filtration units (primary filtration), rinsing the separated crystals with hot water at 200 to 250°C, separating a second mother liquor from the rinsed crystals (secondary filtration), centrifuging the separated crystals to obtain a crystalline powder of 2,6-naphthalene dicarboxylic acid, and drying the crystalline powder of 2,6-naphthalene dicarboxylic acid.

In accordance with another aspect of the present invention, there is provided an apparatus for purifying 2,6-naphthalene dicarboxylic acid crystals obtained after crystallization, comprising sintered metal membrane filtration units for receiving a slurry of 2,6-naphthalenedicarboxylic acid from crystallizers and separating the mother liquor from the crystals, second filtration units for rinsing the separated crystals with hot water at 200 to 250°C and separating a second mother liquor from the crystals, a water reservoir for supplying hot water to the sintered metal membrane filtration units and the second filtration units, a centrifuge for centrifuging the crystals filtered by the second filtration units to provide a crystalline powder of 2,6-naphthalene dicarboxylic acid, and a dryer for drying the crystalline powder of 2,6-naphthalene dicarboxylic acid.

**Advantageous Effects**

According to the method and the apparatus of the present invention, sintered metal filters are used to purify 2,6-naphthalene dicarboxylic acid with a purity as high as 99.9% by weight in a yield as high as 95%. Therefore, the method and the apparatus of the present invention are suitable for the purification of 2,6-naphthalene dicarboxylic acid on an industrial scale. In addition, the use of the sintered metal filters is economically advantageous in terms of maintenance and management.

Furthermore, according to the method and the apparatus of the present invention, since filtration and rinsing operations can be successively carried out after hydrogenation of NDA, the overall procedure, including operations before the hydrogenation, can be carried out in a continuous process. Therefore, the method and the apparatus of the present invention provide advantageous effects in terms of workability.

**Brief Description of the Drawings**

FIG. 1 is a process flow chart illustrating a method for purifying 2,6-naphthalenedicarboxylic acid according to the present invention.

FIG. 2 is a detailed diagram of sintered metal membrane filtration units used in an apparatus for purifying 2,6-naphthalene dicarboxylic acid according to the present invention.

FIG. 3 is a schematic diagram illustrating the separation direction of the mother liquor in one of the sintered metal membrane filtration units of FIG. 2 and the filtration principle of the membrane filtration unit.

FIG. 4 schematically shows the shapes of crystal cakes formed on the sintered metal membrane filtration units of FIG. 2 according to various structures of the membrane filtration units.

FIG. 5 is a detailed view of a sintered metal membrane filter used in a sintered metal membrane filtration unit of a purification apparatus according to the present invention.

FIGs. 6a to 6f are schematic cross-sectional diagrams showing the intervals and arrangements of membrane filters according to the treatment capacity of sintered metal membrane filtration units of a purification apparatus according to the present invention.

FIG. 7 is a graph showing the particle size distribution of NDA crystals purified using a purification apparatus of the present invention, which is used for the optimization of membrane filtration units of the purification apparatus.

Brief explanation of essential parts of the drawings

71, 72: Hot water feed ports 73: Mother liquor discharge port

74: Balance line 75: Crystallized slurry feed port

76: Slurry discharge port 77: Pressure release port

78, 79: Manometers 80: Thermometer

81: Lower N₂ feed port 82: Upper N₂ feed port

**Mode for the Invention**

Preferred embodiments of the present invention will now be described in greater detail with reference to the accompanying drawings.

The present invention provides a purification method of 2,6-naphthalene dicarboxylic acid that uses sintered metal membrane filters to filter and rinsing 2,6-naphthalene dicarboxylic acid from a slurry containing the 2,6-naphthalene dicarboxylic acid so that the 2,6-naphthalene dicarboxylic acid can be recovered in high purity. Particularly, the method of the present invention can be carried out after hydrogenation and crystallization in a continuous process.

According to the method of the present invention, impurities dissolved in 2,6-naphthalene dicarboxylic acid crystals produced after hydrogenation and crystallization are removed in accordance with the following procedure. First, sintered metal membrane filtration units are used to separate mother liquor from the crystals (primary filtration). Subsequently, the separated crystals are rinsed with hot water at 200 to 250°C. The hot water is fed into the sintered metal membrane filtration units to prepare a slurry of the crystals. Then, the slurry is further rinsed with hot water at 200 to 250°C.

A second mother liquor is separated from the rinsed crystals (secondary filtration). Thereafter, the separated crystals are subjected to centrifugation to obtain a crystalline powder of high-purity 2,6-naphthalene dicarboxylic acid, after which the crystalline powder is dried.

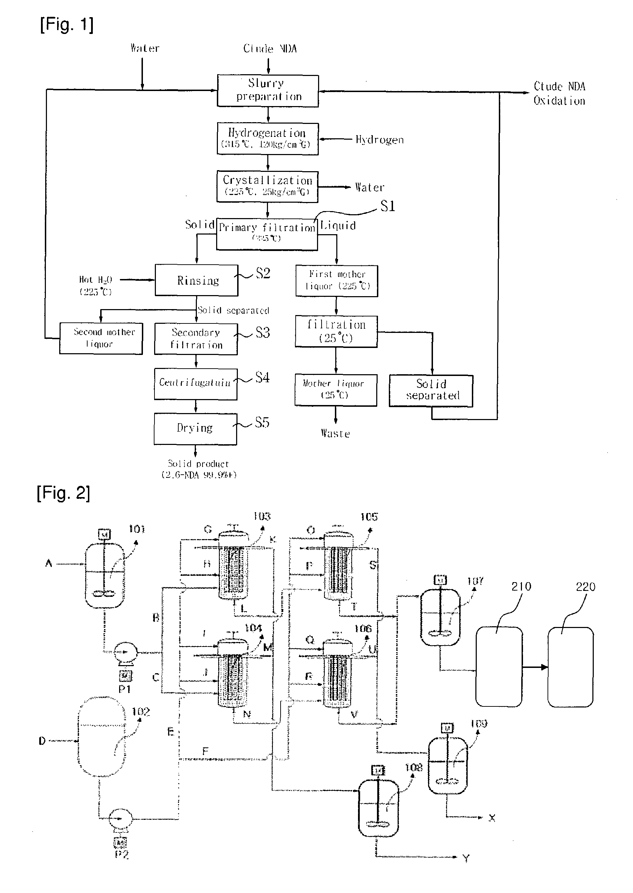

A more detailed explanation of the method according to the present invention will be provided below with reference to FIG. 1. The purification method of 2,6-naphthalene dicarboxylic acid consists of hydrogenation, crystallization, filtration/rinsing, solid- liquid separation, and drying. The filtration and rinsing step is carried out under high-temperature and high-pressure conditions to discharge mother liquors containing impurities and solvents.

The purification of 2,6-naphthalene dicarboxylic acid by hydrogenation is performed by the following procedure. Crude 2,6-naphthalene dicarboxylic acid is mixed with a solvent used for hydrogenation in a certain ratio in a slurry preparation tank to prepare a homogeneous slurry. The solvent is preferably water or acetic acid. The amount of the solvent used is not particularly limited so long as 2,6-naphthalene dicarboxylic acid can be homogeneously present under the temperature and pressure conditions for hydrogenation. The internal temperature of the slurry preparation tank is maintained constant to shorten the time required to prepare the homogeneous slurry and considerably improve the homogeneity of the slurry. The temperature of the slurry preparation tank is maintained at 40-150°C, preferably at 60-100°C.

The slurry is passed through a pre-heater to be heated to a hydrogenation temperature at which the 2,6-naphthalene dicarboxylic acid is substantially dissolved in the solvent. The hot slurry is passed through a heat exchanger for secondary dissolution, and is then introduced into a hydrogenation reactor. The heat exchanger serves to dissolve a slight amount of the 2,6-naphthalene dicarboxylic acid that remains undissolved and to remove impurities ( e.g., other organic materials and metal components) insoluble in the solvent. As a result, the 2,6-naphthalene dicarboxylic acid is in a completely homogeneous state in the heat exchanger. It is preferred that the temperature and pressure conditions of the heat exchanger be the same as or slightly higher than those for subsequent hydrogenation.

The solution passing through the heat exchanger is fed into a hydrogenation reactor containing a hydrogenation catalyst (e.g., Pd/C or Pt/C) and hydrogenated with hydrogen at 290-350°C to remove impurities (e.g., FNA and Br-NDA) or convert the impurities to readily removable forms. The amount of the hydrogen fed into the hydrogenation reactor is selectively determined depending on the amounts and reactions of impurities contained in the crude 2,6-naphthalene dicarboxylic acid.

The 2,6-naphthalene dicarboxylic acid passing through the hydrogenation reactor is crystallized in multi-stage crystallizers, preferably four crystallizers, where stepwise drops in crystallization pressure and temperature occur to crystallize the purified 2,6-naphthalene dicarboxylic acid. For the crystallization of the NDA, the temperatures of the crystallizers are lowered at a rate of 30°C/min or less to an optimal filtration temperature. The optimal temperature is referred to a temperature at which the 2,6-naphthalene dicarboxylic acid is precipitated as much as possible while reaction by-products are still dissolved in the solvent. The optimal temperature is typically in the range of 150 to 250°C.

The crystallization through the four-stage pressure and temperature drops allows the2,6-naphthalene dicarboxylic acid crystals to be present in a slurry state because the solubility of the 2,6-naphthalene dicarboxylic acid is low (0.5% at 225°C) under high- temperature and high-pressure conditions [e.g., 225°C, 2'451'663 N/m²(25 kg/cm²)]. Most other impurities are dissolved in the solvent.

Few equipment and apparatuses capable of continuously separating crystals and mother liquor under high-temperature and high-pressure conditions have been developed to date. Under these circumstances, the present invention provides a method for filtering and rinsing the NDA-containing slurry to recover NDA in high purity.

The filtration is performed at the same temperature as that of the crystallizers and under pressure to recover a sufficient amount of pure NDA. Referring to FIG. 1, sintered metal membrane filtration units are used to separate a first mother liquor from the 2,6-naphthalene dicarboxylic acid crystals obtained after the crystallization (Step S1). The first mother liquor is rapidly cooled to room temperature and crystallized to be separated into mother liquor and a solid component. The separated mother liquor is filtered and discarded. A portion of the separated solid component is sent back to the slurry preparation tank and the remaining portion thereof is transferred to an oxidation reactor, which is disposed upstream of the hydrogenation reactor.

A specified amount of hot water at 200 to 230°C is transferred to rinse the crystals filtered by the sintered metal membrane filtration units. Hot water is fed into the sintered metal membrane filtration units to prepare a slurry of the crystals. Then, the slurry is further rinsed with hot water. After the hot water rinsing, secondary filtration is performed to separate second the mother liquor from the crystals.

The second mother liquor can be circulated into the slurry preparation tank, which is disposed upstream of the hydrogenation reactor. The separated solids are transferred to a centrifuge, where hot water used for the transfer is used to further rinse the solids.

The centrifugation gives a crystalline powder of 2,6-naphthalene dicarboxylic acid.The crystalline powder is dried in a dryer at a temperature of 110-150°C to recover 2,6-naphthalene dicarboxylic acid in a solid state in a purity of at least 99.9% by weight.

In another aspect, the present invention provides an apparatus for purifying 2,6-naphthalene dicarboxylic acid that is present in a solid state in a solution. FIG. 2 is a schematic diagram of an apparatus for purifying 2,6-naphthalene dicarboxylic acid according to an embodiment of the present invention. A detailed explanation of the purification apparatus according to the present invention will be given below with reference to FIG. 2.

Referring to FIG. 2, the purification apparatus of the present invention comprises sintered metal membrane filtration units for receiving a slurry of 2,6-naphthalene dicarboxylic acid crystals and a mother liquor from crystallizers and separating the mother liquor from the crystals, second filtration units for rinsing the separated crystals with hot water and separating a second mother liquor from the crystals, a water reservoir for supplying hot water to the sintered metal membrane filtration units and the second filtration units, a centrifuge for centrifuging the crystals filtered by the second filtration units to provide a crystalline powder of 2,6-naphthalene dicarboxylic acid, and a dryer for drying the crystalline powder of 2,6-naphthalene dicarboxylic acid.

The apparatus of the present invention may further comprise a first mother liquor reservoir connected to the sintered metal membrane filtration units to store the mother liquor separated by solid- liquid separation, a rinsing mother liquor reservoir connected to the second filtration units to store the rinsing mother liquor from the second filtration units, and a reservoir disposed upstream of the centrifuge.

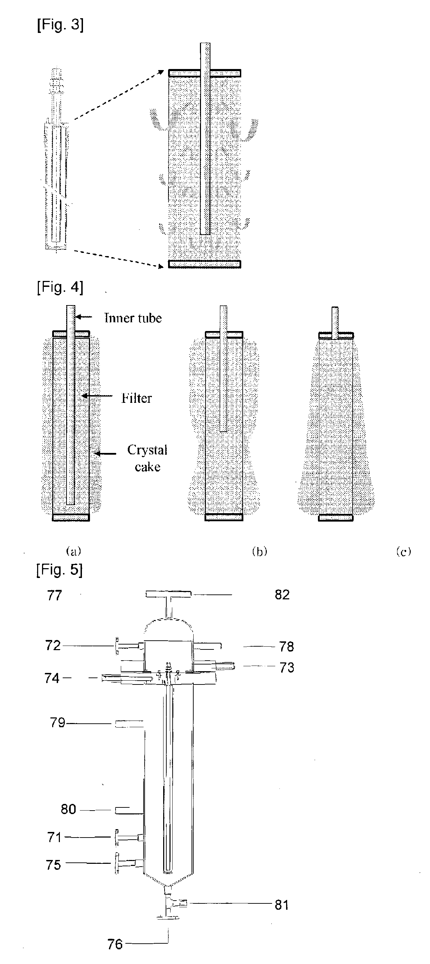

FIG. 5 shows one of the sintered metal membrane filtration units. The sintered metal membrane filtration unit includes two ports through which the hot solvent is fed, a port through which the mother liquor filtrate is discharged, a balance line for balancing the pressures of the introduced solvent and the transferred slurry, a port through which the crystallized slurry is introduced, a port through which a mixture of the separated crystalline powder and the solvent is discharged, a port through which gas and steam are evolved or the pressure releases, manometers for controlling the performance and the pressure of the membrane, a thermometer for measuring the internal temperature of the unit, and pressurization ports for controlling the internal pressure of the unit.

The two solvent (e.g., water) feed ports are formed at upper and lower portions of the sintered metal membrane filtration unit, respectively. The bottom of the sintered metal membrane filtration unit may be tapered to facilitate the transfer of the slurry. The slurry discharge port is formed at a lower end of the tapered bottom. One of the pressurization ports is formed at a lower end portion of the tapered bottom to control the internal pressure of the unit. The other pressurization port is formed at an uppermost end of the unit to control the internal pressure of the unit. The gas and steam or pressure release port is also formed at an uppermost end of the unit. The balance line is connected to the reservoirs of the previous stages (i.e. the fourth crystallizer for the first filtration unit and the first filtration/rinsing unit for the second filtration unit) to be responsible for the adjustment of the pressure balance of the introduced solvent and the transferred slurry. The crystallized slurry feed port is formed under the lower solvent introducing port and the thermometer is disposed above the lower solvent feed port to measure the internal temperature of the unit.

The sintered metal membrane filtration unit includes an inner tube having a length similar to that of the filter. The sintered metal membrane filtration unit may include a plurality of membrane filters made of stainless steel, a corrosion resistant alloy or a heat resistant alloy and having a thickness of 0.1 to 1 cm. The pores of the membrane filters may have pores with a diameter ranging from 0.1 µm to 5µm.

Hereinafter, the operation of the purification apparatus according to the present invention will be explained.

A slurry of crystals and a mother liquor is transferred at a flow rate of 45 kg/hr to a fourth crystallizer 101 (Flow A) by a pressure difference between a third crystallizer [pressure: 4'412'993 N/m²(45 kg/cm²), temperature: 225°C] and the fourth crystallizer [pressure: 2'451'663 N/m²(25 kg/cm²), temperature: 215°C]. The slurry is transferred to sintered metal membrane filtration units 103 and 104, where the mother liquor n is separated, by means of a slurry transfer pump Pl. The transfer of the slurry to the sintered metal membrane filtration units 103 and 104 is continuously repeated at intervals of 10-20 minutes. The continuous transfer is controlled by an automatic controller. The transfer intervals are varied depending on the capacity of rinsing units and the performance of the filters. The inner temperature of first sintered metal membrane filtration units is maintained at 200-250°C and the inner pressure is 1'961'330-4'903'325 N/m²(20-50 kg/cm²) .

The transfer of the slurry along a line C is stopped while the slurry is transferred along a line B. That is, the slurry is filtered in the sintered metal membrane filtration unit 104 while the slurry is transferred to the sintered metal membrane filtration unit 103. As a result, a crystalline powder is present in the sintered metal membrane filtration unit 104 and the crystals adhered to the surfaces of the sintered metal filter are again dispersed with water transferred by means of a water transfer pump P2 for rinsing. The dispersion is transferred to a second filtration unit 106 (Flow N).

After completion of the transfer to the sintered metal membrane filtration unit 103, the sintered metal membrane filtration unit 103 enters a mother liquor filtration mode and the sintered metal membrane filtration unit 104 receives new slurry from the crystallizer 101.

The transfer of the slurry to the sintered metal membrane filtration units 103 and 104 is repetitively manipulated at intervals of 10-20 minutes by an automatic controller. Water is transferred to the sintered metal membrane filtration units 103 and 104 along lines I, J, G and H. The water flows G and I are intermittently conducted by automatic control to take off the crystalline powder adhered to the sintered metal filters. Water is also transferred along lines H and J. The slurry transferred to the second filtration units (or rinsing tanks) 105 and 106 is filtered in the same manner as in the primary filtration. The inner temperature of second sintered metal membrane filtration units is maintained at 200-250°C and the inner pressure is 1'961'330-4'903'325 N/m²(20-50 kg/cm²).

Water is supplied along a line F to the second filtration units 105 and 106 to take off the crystalline powder adhered to the second filtration units 105 and 106. The filtered crystals are transferred to a reservoir 107 disposed upstream of a centrifuge 210 along lines T and V by automatic control. The crystals stored in the reservoir 107 are transferred to the centrifuge 210, followed by centrifugation to provide a crystalline powder of 2,6-naphthalenedicarboxylic acid. The crystalline powder is dried in a dryer 220.

The mother liquor separated from the first filtration unit is transferred to a mother liquor reservoir 108 and is again subjected to solid-liquid separation by a temperature drop to room temperature and a pressure drop (Flow Y). The subsequent procedure is the same as explained previously. The rinsing mother liquor used for the secondary filtration is transferred to a rinsing mother liquor reservoir 109 through flows S and U and is transferred to a slurry preparation tank via a line X.

The filtration steps are carried out at a pressure of 1'961'330-4'903'325 N/m²(20 to 50 kg/cm²). Specifically, the pressure of the mother liquor reservoir is controlled to reach a pressure difference of 196'133-2'451'663 N/m² (2 to 25 kg/cm²) . at which the filtration is performed. At a pressure difference smaller than 196'133N/m²(2 kg/cm²), the filtration is performed at a low rate in view of the characteristics of the sintered metal membrane filtration units, thus requiring much operating time. Meanwhile, there exists a danger of damage to the metal filters at a pressure difference larger than 2'451'663 N/m (25 kg/cm²_{) .}

According to the purification apparatus of the present invention, NDA can be separated in a purity of at least 99.9% by weight and a yield of at least 95% by using the sintered metal membrane filters as high-pressure rinsing units.

Since the sintered metal membrane filters can withstand high temperatures as well as low temperatures and are highly resistant to thermal impact, they can be used at places where sudden temperature variations occur. In addition, since the sintered metal membrane filters do not substantially absorb or react with medium components that are in contract with them, they are suitable for use in reactors under corrosive environment. Furthermore, since the sintered metal membrane filters are highly resistant to mechanical impact, they are advantageously used in places susceptible to high pressure or vibration.

The cleaning principle of the sintered metal filters used in the purification apparatus of the present invention will be explained below with reference to FIG. 3. The mother liquor and cleaning solution such as NaOH solution are filtered and cleaned through the membrane filter of the sintered metal filter as indicated by arrows in FIG. 3. A tube having a predetermined length is formed within the rinsing and cleaning filter to assist in the filtration of the mother liquor and cleaning solution over the entire surface of the filter. As shown in FIGs. 4a, 4b and 4c, when an inner tube is relatively short, cakes of the crystalline powder are not uniformly formed on the filter, resulting in a deterioration in the performance of the filter. A sufficiently high efficiency of the filter is attained only when the inner tube extends substantially to the bottom of the filter.

The interval between the inner tube and the filter, the diameter of the filter, the length of the inner tube and the length of the filter are considered as correlative factors for attaining sufficiently high efficiency of the filter. The length of the filter may vary depending on the treatment capacity of the filter. It is prefer that the filter be 50 cm to 2 m in length. The inner tube has a length that extends substantially to the bottom of the filter. When the filter has a length of 50 cm to 2 m, the length of the inner tube is generally 0.5 to 2 cm shorter than that of the filter. Preferably, the filter has a diameter of 10 to 50 cm, a thickness of 0.1 to 1 cm and a pore size of 0.1 to 50 µm.

The sintered filters are porous high-strength products taking advantage of the inherent porosity of powder metallurgy. An optimal combination of elemental techniques is significant in the production of the sintered filters. The use of general mesh filters in place of the sintered filters causes a relatively low yield and clogging of the mesh pores, resulting in deterioration of filtering performance after long-term use.

In contrast, since back flushing can be simultaneously conducted in the unit processing when the sintered metal filters are used, a deterioration in the filtering performance of the sintered metal filters is negligible. Although the filtering performance of the sintered metal filters is deteriorated after long-term use, cleaning of the sintered metal filters with an aqueous NaOH solution can maintain the initial filtering performance of the filters.

Since the sintered metal filters have a three-dimensional complex porous structure, they exhibit higher filtering efficiency than two-dimensional mesh filters. In addition, the sintered metal filters have a very high degree of freedom in shape and a relatively long service time even under extreme conditions. Furthermore, the sintered metal filters are efficient due to their ease of cleaning. Moreover, since the sintered stainless steel filters exhibit excellent characteristics in terms of corrosion resistance, heat resistance and stiffness, they can be advantageously used under high-temperature and high-pressure conditions.

Sintered metal filters are largely divided into two kinds, i.e. sintered metal fibrous membrane filters and sintered metal powder membrane filters. Depending on the size or diameter variation of the powder, the porosity of the filter materials may vary in a wide range of 10% to 95%. However, the generally applicable porosities of sintered metal fibrous membrane filters and sintered metal powder membrane filters vary in the range of 60-90% and 30-40%, respectively. Bronze and stainless steel powders are mainly used as materials for the metal powder filters, and nickel, titanium and Inconel powders are also used to manufacture filters for special applications. The sintered metal filters used in the present invention are made of stainless steel (e.g., SUS316L), highly corrosion resistant alloys (e.g., Hastelloy/Carpenter 20CB3), heat resistant alloys (e.g., INCONEL 601/Nickel 200), and the like. The maximum applicable temperature and pressure are 500°C and 9'806'650 N/m²(100 kg/cm²), respectively.

FIG. 5 is a detailed view of one of the sintered metal membrane filtration units of the purification apparatus according to the present invention. In FIG. 5, numerals 71 and 72 denote ports through which the solvent at 200-250°C is transferred, numeral 73 denotes a port through which the mother liquor filtrate is discharged, numeral 74 denotes a balance line for balancing the pressures of the fed solvent and the transferred slurry, mineral 75 denotes a port through which the crystallized slurry is fed, numeral 76 denotes a port through which a mixture of the separated crystalline powder and the solvent is discharged, mineral 77 denotes a port through which gas and steam are evolved or the pressure releases, numerals 78 and 79 denote manometers for controlling the performance and the pressure of the membrane, mineral 80 denotes a thermometer for measuring the internal temperature of the unit, and numerals 81 and 82 denote N₂ feed ports for controlling the internal pressure of the unit [72] The treatment capacity of the sintered metal membrane filtration units is determined depending on various parameters (e.g., porosity per unit area) of the filters.

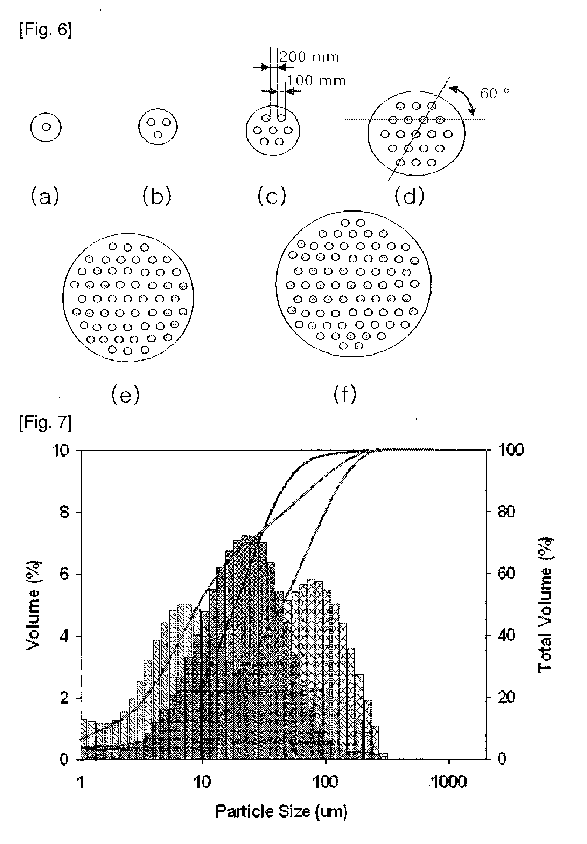

FIG. 6 shows various structures of the membrane filters according to the capacity of the rinsing units. As shown in FIG. 6, the arrangements of one or more membrane filters are maintained constant to prevent crystal cakes from being adhered between the membrane filters and to maximize the separation efficiency of the membrane filters. The treatment capacities of the sintered metal membrane filtration units according to the number of the filters are summarized in Table 1.

Table 1

**[Table 1]**

| | (a) | (b) | (c) | (d) | (e) | (f) |
|---|---|---|---|---|---|---|
| Number of filters | 1 | 3 | 7 | 19 | 55 | 73 |
| Treatment capacity (kg/hr)@ of 0.3 µm filters | 600 | 1, 800 | 4,200 | 11,400 | 33,000 | 43,800 |
| Treatment capacity (kg/hr) of 0.5 µm filters | 1,000 | 3,000 | 7,000 | 19, 000 | 55,000 | 73,000 |

| | | | | | | |
|---|---|---|---|---|---|---|
| @slurry concentration = NDA 10%, the average particles size of crystals was 50µm | | | | | | |

The membrane filters must be arranged in such a manner that they are maintained at constant intervals depending on the thickness of crystal cakes. It is preferred to use membrane filters having a diameter of 100 mm and a pore size of 0.1-50µm, preferably 0.3-5 µm, in terms of maximum filtering efficiency. The preferred pore size (0.3-5 µm) was determined based on the particle size distribution of NDA crystals shown in FIG. 7 taking into consideration the yield and cleaning of the NDA crystals. That is, the size and the particle size distribution of the crystals are substantially determined during crystallization, but it is a need to optimize the membrane filters depending on the applications and characteristics of the products.

The final NDA samples obtained using the membrane filters having a pore size of 0.1 to 10 µm were collected, and analyzed for average crystal size and particle size distribution using a particle size analyzer, which is used for the measurement of samples having a size of 0.1 to 1,000 µm. FIG. 7 shows the particle size distribution of the NDA crystals separated through the sintered metal membrane filtration units after controlled crystallization. The graph of FIG. 7 shows that the NDA crystals had a particle size distribution of 1 to 350 µm and an average crystal size of 10 to 100 µm.

Hereinafter, the present invention will be explained in more detail with reference to the following examples. However, the following examples serve to provide further appreciation of the invention but are not meant in any way to restrict the scope of the invention.

**EXAMPLES**

**Example 1**

A slurry of 2,6-naphthalene dicarboxylic acid crystals as a raw material was transferred from a fourth crystallizer at 2'451'663N/m²(25 kg/cm²)and 215°C to 60 L sintered metal membrane filtration units by means of a pump. The slurry had a concentration of 10% and a volume of 60 L. As soon as the slurry was transferred, the filtration of the slurry was performed for 5 minutes. After completion of the transfer, the filtration was further performed for 3 minutes. As a result, cakes of 2,6-naphthalene dicarboxylic acid were formed on tubes of the sintered metal membrane filtration units and the mother liquor was completely separated. While the separation was performed in one of the sintered metal membrane filtration units, another raw material was transferred to the other sintered metal membrane filtration unit. After completion of the separation, 60 L of hot rinsing water at 220°C was transferred to the filtration units for one minute. After the transfer was finished, the slurry was transferred to second filtration units for one minute. The overall procedure was repetitively conducted in the two sintered metal membrane filtration units at intervals of 10 minutes. The sintered metal membrane filtration units included membrane filters having a pore size of 0.5 µm. The slurry transferred to the second filtration units were separated in the second sintered metal membrane filtration units. The transfer and the filtration of the slurry were continued for 5 minutes. The secondary rinsing was finished, and then water was used to send the rinsed crystals to the next stage (i.e. centrifugation).

To evaluate the separation efficiency of the filtrations units, samples were collected from the fourth crystallizer, after the primary filtration and after the secondary rinsing, and analyzed by displacement gas chromatography (GC). The results are shown in Table 2.

Table 2

**[Table 2]**

| | | From crystallizer | After primary filtration | After secondary filtration |
|---|---|---|---|---|
| | | | Purity (wt%) | |
| Components | 2,6-NDA | 99.5115 | 99.8547 | 99.9522 |
| | 2,6 -FNA | 0 0004 | 0 0000 | 0 0000 |
| | 2-NA | 0 2409 | 0 0490 | 0 0000 |
| | MNA | 0.0173 | 0.0091 | 0.0000 |
| | TMLA | 0 1133 | 0 0672 | 0 0386 |
| | Br-NDA | 0 0003 | 0 0000 | 0 0000 |
| | DCT | 0 0610 | 0 0000 | 0 0000 |
| | Undefined | 0 0553 | 0 0200 | 0 0092 |
| Solid, content (kg) | | 6 kg | - | - |
| Yield (kg) | | - | 5.81 | 5.73 |
| Yield (%) | | - | 97.16 | 98.71 |
| Total yield (%) | | | 95.92 | |

**Comparative Example 1**

In this example, the apparatus disclosed in Korean Unexamined Patent Publication No. 2005-0064022 was used. First, 336 kg of a slurry solution containing 36kg of a solid was introduced from a slurry supply unit to a 400 L meshed rinsing unit equipped with a filter, an agitator and an inner valve while maintaining the pressure with N₂. The filtrate was discharged to a high-pressure filtrate recovery unit through a filtrate discharge port, and the solids were filtered through the filter. After the discharged first filtrate was transferred to a low-pressure filtrate recovery unit, the pressure was dropped to ambient pressure to discard the filtrate. The internal temperature of the filtrate rinsing unit was adjusted to 225°C and the mesh size of the filter was set to 20 µm. The pressure of the filtrate rinsing unit was maintained at 2'549'729 N/m²(26 kg/cm²) using nitrogen and that of the high-pressure filtrate recovery unit was maintained constant with N₂ supplied through one side of the recovery unit. The pressure of the filtrate recovery unit was 49'033.25-196'133 N/m²(0.5-2 kg/cm²) lower than that of the filtrate rinsing unit. Next, 300 kg or more of a preheated solvent (> 200°C) was fed from a solvent heating supply unit to the rinsing unit and stirred with an anchor type agitator in 80 rpm for 30 minutes. The second filtrate was discharged to the high-pressure filtrate recovery unit. The rinsing was repeated once more. 100 kg of a solvent (> 200°C) was supplied from the solvent heating supply unit to the rinsing unit and stirred to prepare a slurry containing NDA. The slurry was sent to a high-pressure slurry recovery unit along a slurry discharge line. The pressure of the high-pressure slurry recovery unit was maintained constant with N₂ supplied through one side of the recovery unit. The pressure of the slurry recovery unit was equal to or 49'033.25-196'133 N/m²(0.5-2 kg/cm²) lower than that of the slurry rinsing unit. After the slurry transferred to the high-pressure slurry recovery unit was transferred to the low-pressure slurry recovery unit, the pressure was dropped to ambient pressure. The solvent was removed to recover pure NDA. The solids thus formed and contents thereof are shown in Table 3.

Table 3

**[Table 3]**

| Organic materials | NDA | NA | MNA | DCT | Others |
|---|---|---|---|---|---|
| Amount (kg) | 33.585 | 0.014 | 0.029 | 0.001 | 0.017 |
| Content (%) | 99.818 | 0.042 | 0.086 | 0.003 | 0.051 |

Example 2

In this example, the kind of the sintered metal filters was varied to compare the performance of the filters. The performance of the sintered metal filters (0.1, 0.3, 0.5, 1 and 10 µm) was analyzed in the same procedure as described in Example 1. The analytical results are shown in Table 4.

Table 4

**[Table 4]**

| | | 0.1 µm filter | 0.3µm filter | 0.5µm filter | 1µm filter | 10µm filter |
|---|---|---|---|---|---|---|
| 2, 6-NDA | 99.2699 | 99. 9021 | 99.9522 | 99.9730 | 99. 9855 | |
| 2, 6-FNA | 0.0002 | 0.0000 | 0.0000 | 0.0000 | 0.0000 | |
| 2-NA | 0.0207 | 0.0168 | 0.0000 | 0.0000 | 0.0000 | |
| MNA | 0.0073 | 0.0081 | 0.0000 | 0.0000 | 0.0000 | |
| TMLA | 0.0833 | 0.0572 | 0.0386 | 0.0213 | 0.0145 | |
| Br-NDA | 0.0003 | 0.0000 | 0.0000 | 0.0000 | 0.0000 | |
| DCT | 0.0030 | 0.0000 | 0.0000 | 0. 0000 | 0.0000 | |
| Undefined | 0.0153 | 0.0158 | 0.0092 | 0. 0056 | 0.0000 | |
| Yield(%) | | 98.10 | 97.16 | 95.92 | 91.32 | 85. 24 |

The results of Table 4 lead to the conclusion that organic impurities, such as NA, MNA and DCT, can be readily removed within a short time and the purity of the final product can be increased above 99.8% by the method of the present invention.

[96]

## Claims

1. A method for purifying 2,6-naphthalene dicarboxylic acid, the method
comprising:
(a) a primary filtration step
separating a first mother liquor from 2,6-naphthalene dicarboxylic acid crystals obtained after crystallization using sintered metal membrane filtration units;
(b) a first rinsing step
rinsing the separated crystals with hot water at 200-250°C;
(c) a secondary filtration step
separating a second mother liquor from the rinsed crystals;
(d) a second rinsing step
rinsing the separated crystals with hot water at 200-250°C;
(e) an isolation step
centrifuging the separated crystals to obtain a crystalline powder of
2,6-naphthalene dicarboxylic acid; and
(f) a dry step
drying the crystalline powder of 2,6-naphthalene dicarboxylic acid.

2. The method according to claim 1, wherein the first and second filtration and first and second rinsing are carried out at an internal temperature of 200 to 250°C and a pressure of 1'961'330 to 4'903'325 N/m²(20 to 50 kg/cm²).

3. The method according to claim 1, wherein the filtration is carried out using a pressure difference of 196'133 to 2'451'663 N/m²(2 to 25 kg/cm²) .

4. The method according to claim 1, wherein the sintered metal membrane filtration units include membrane filters made of stainless steel, a corrosion resistant alloy or a heat resistant alloy and having a pore size of 0.1 µm to 5 µm.

5. The method according to claim 1, wherein the final 2,6-naphthalene dicarboxylic acid crystals have an average crystal size of 10 to 100 µm.

6. An apparatus for purifying 2,6-naphthalene dicarboxylic acid crystals obtained after crystallization, comprising
(a) first sintered metal membrane filtration units for receiving a slurry of 2,6-naphthalene dicarboxylic acid crystals and a mother liquor from crystallizers and separating the mother liquor from the crystals,
(b) second sintered metal membrane filtration units for rinsing the separated crystals with hot water and separating a second mother liquor from the crystals,
(c) a water reservoir for supplying hot water to the sintered metal membrane filtration units and the second filtration units,
(d) a centrifuge for centrifuging the crystals filtered by the second filtration units to provide a crystalline powder of 2,6-naphthalene dicarboxylic acid, and
(e) a dryer for drying the crystalline powder of 2,6-naphthalene dicarboxylicacid.

7. The apparatus according to claim 6, further comprising a first mother liquor reservoir connected to the sintered metal membrane filtration units to store the mother liquor separated by solid- liquid separation, a rinsing mother liquor reservoir connected to the second filtration units to store the rinsing mother liquor from the second filtration units, and a reservoir disposed upstream of the centrifuge.

8. The apparatus according to claim 6, wherein each of the sintered metal membrane filtration units includes two ports through which the hot solvent at 200 to 250°C is fed, a port through which the mother liquor filtrate is discharged, a balance line for balancing the pressures of the introduced solvent and the transferred slurry, a port through which the crystallized slurry is fed, a port through which a mixture of the separated crystalline powder and the solvent is discharged, a port through which gas and steam are evolved or the pressure releases, manometers for controlling the performance and the pressure of the membrane, a thermometer for measuring the internal temperature of the unit, and pressurization ports for controlling the internal pressure of the unit.

9. The apparatus according to claim 6, wherein each of the sintered metal membrane filtration units includes a filter having a length of 50 cm to 2 m, a diameter of 10 to 50 cm and a thickness of 0.1 to 1 cm and an inner tube whose length extends substantially to the bottom of the filter and is 0.5 to 2 cm shorter than that of the filter.

10. The apparatus according to claim 6, wherein the sintered metal membrane filtration units include membrane filters made of stainless steel, a corrosion resistant alloy or a heat resistant alloy and having a pore size of 0.1 µm to 5 µm.

## Patentansprüche

1. Ein Verfahren zur Reinigung von 2,6-Naphthalindicarbonsäure, wobei das Verfahren Folgendes umfasst:
(a) einen ersten Filtrationsschritt
Separieren einer ersten Mutterlauge aus 2,6-Naphthalindicarbonsäure-Kristallen erhalten durch eine Kristallisation unter Verwendung von Sintermetall-Membranfiltrationseinheiten;
(b) einen ersten Spülschritt
Spülen der abgetrennten Kristalle mit heissem Wasser bei 200-250 °C;
(c) einen zweiten Filtrationsschritt
Separieren einer zweiten Mutterlauge aus den gespülten Kristallen;
(d) einen zweiten Spülschritt
Spülen der abgetrennten Kristalle mit heissem Wasser bei 200-250 °C;
(e) einen Isolierungsschritt
Zentrifugieren der abgetrennten Kristalle um ein kristallines 2,6-Naphthalindicarbonsäure-Pulver zu erhalten; und
(f) einen Trocknungsschritt
Trocknen des kristallinen 2,6-Naphthalindicarbonsäure-Pulvers.

2. Verfahren nach Anspruch 1, wobei das erste und zweite Filtrieren und das erste und zweite Spülen bei einer Innentemperatur von 200 bis 250 °C und einem Druck von 1'961'330 bis 4'903'325 N/m ² (20 bis 50 kg/cm²) durchgeführt werden.

3. Verfahren nach Anspruch 1, wobei das Filtrieren bei einer Druckdifferenz von 196'133 bis 2'451'663 N/m² (2 bis 25 kg/cm²) durchgeführt wird.

4. Verfahren nach Anspruch 1, wobei die Sintermetall-Membranfiltrationseinheiten Membranfilter aus Edelstahl, einer korrosionsbeständigen Legierung oder einer hitzebeständigen Legierung und mit einer Porengrösse von 0.1 µm bis 5 µm umfassen.

5. Verfahren nach Anspruch 1, wobei die endgültigen 2,6-Naphthalindicarbonsäure-Kristalle eine mittlere Kristallgröße von 10 bis 100 µm aufweisen.

6. Eine Vorrichtung zum Reinigen von 2,6-Naphthalindicarbonsäure-Kristallen, welche durch Kristallisation erhalten worden sind, umfassend
(a) erste Sintermetall-Membranfiltrationseinheiten zur Aufnahme einer Aufschlämmung aus 2,6-Naphthalindicarbonsäure-Kristallen und einer Mutterlauge aus Kristallisatoren und Separierung der Mutterlauge von den Kristallen,
(b) zweite Sintermetall-Membranfiltrationseinheiten zum Spülen der separierten Kristalle mit heißem Wasser und Separierung einer zweiten Mutterlauge von den Kristallen,
(c) ein Wasser-Reservoir zum Zuführen von heissem Wasser zu den Sintermetall-Membranfiltrationseinheiten und den zweiten Filtrationseinheiten,
(d) eine Zentrifuge zum Zentrifugieren der durch die zweiten Filtrationseinheiten gefilterten Kristalle, um ein kristallines 2,6-Naphthalindicarbonsäure-Pulver zu erhalten, und
(e) einen Trockner zum Trocknen des kristallinen 2,6-Naphthalindicarbonsäure-Pulvers.

7. Vorrichtung nach Anspruch 6, ferner aufweisend ein erstes Mutterlaugenreservoir verbunden mit den Sintermetall-Membranfiltrationseinheiten, um die durch Fest-Flüssig-Trennung separierte Mutterlauge aufzubewahren, ein Mutterlaugenspülreservoir verbunden mit den zweiten Sintermetall-Filtereinheiten, um die Spülflüssigkeit der Mutterlauge aus den zweiten Filtrationseinheiten aufzubewahren, und ein der Zentrifuge vorgelagertes Reservoir.

8. Vorrichtung nach Anspruch 6, wobei jede der Sintermetall-Membranfiltrationseinheiten zwei Öffnungen aufweist, durch welche das heisse Lösungsmittel bei einer Temperatur von 200 bis 250 °C zugeführt wird, eine Öffnung, durch welche das Mutterlaugenfiltrat abgeführt wird, eine Ausgleichsleitung zum Druckausgleich des zugeführten Lösungsmittels und der weitergeleiteten Aufschlämmung, eine Öffnung über welche die kristallisierte Aufschlämmung geführt wird, eine Öffnung über welche ein Gemisch aus separiertem kristallinen Pulver und Lösungsmittel abgeführt wird, eine Öffnung, durch die sich entwickeltes Gas und Dampf oder Druck entweicht, Manometer zur Steuerung der Leistung und des Drucks der Membran, einem Thermometer zur Messung der Innentemperatur der Einheit, und Druckbeaufschlagungsöffnungen zum Steuern des Innendrucks der Einheit.

9. Vorrichtung nach Anspruch 6, wobei jede der Sintermetall-Membranfiltrationseinheiten einen Filter mit einer Länge von 50 cm bis 2 m, einem Durchmesser von 10 bis 50 cm und einer Dicke von 0,1 bis 1 cm und einem inneren Rohr umfasst, dessen Länge sich im Wesentlichen über den Boden des Filters erstreckt und 0,5 bis 2 cm kürzer als die des Filters ist.

10. Vorrichtung nach Anspruch 6, wobei die Sintermetall-Membranfiltrationseinheiten Membranfilter aus Edelstahl, einer korrosionsbeständigen Legierung oder einer hitzebeständigen Legierung und mit einer Porengrösse von 0.1 µm bis 5 µm umfassen.

## Revendications

1. Procédé pour la purification d'acide naphthalène-2,6-dicarboxylic, le procédé comprenant :
(a) une étape primaire de filtration
séparant une première liqueur mère des cristaux d'acide naphthalène-2,6-dicarboxylic obtenus après cristallisation, par l'utilisation des unités de filtration à membrane métallique frittée;
(b) une première étape de rinçage
rinçant les cristaux séparés avec de l'eau chaude à 200-250°C ;
(c) une étape de filtration secondaire
séparant une seconde liqueur mère des cristaux rincés ;
(d) une seconde étape de rinçage
rinçant les cristaux séparés avec de l'eau chaude à 200-250°C ;
(e) une étape d'isolement
centrifugeant les cristaux séparés afin d'obtenir une poudre cristalline d'acide naphthalène-2,6-dicarboxylic ; et
(f) une étape sèche
séchant la poudre cristalline d'acide naphthalène-2,6-dicarboxylic.

2. Procédé selon la revendication 1, dans lequel la première et la seconde filtration et le premier et le second rinçage sont effectués à une température interne de 200 à 250°C et à une pression de 1.961.330 à 4.903.325 N/m² (20 à 50 kg/cm²).

3. Procédé selon la revendication 1, dans lequel la filtration est effectuée en utilisant une différence de pression de 196.133 à 2.451.663 N/M² (2 à 25kg/cm²).

4. Procédé selon la revendication 1, dans lequel les unités de filtration à membrane métallique frittée comprennent des filtres à membrane en acier inoxydable, en un alliage résistant à corrosion, ou en un alliage résistant à la chaleur et ayant une taille de pores de 0.1 µm à 5 µm .

5. Procédé selon la revendication 1, dans lequel les cristaux finaux d'acide naphthalène-2,6-dicarboxylic présentent une taille moyenne des cristaux de 10 à 100 µm.

6. Appareil pour la purification des cristaux d'acide naphthalène-2,6-dicarboxylic obtenus après cristallisation, comprenant
(a) des premières unités de filtration à membrane métallique frittée pour recevoir une suspension épaisse des cristaux d'acide naphthalène-2,6-dicarboxylic et une liqueur mère des cristallisateurs et pour séparer la liqueur mère des cristaux,
(b) des secondes unités de filtration à membrane métallique frittée pour rincer les cristaux séparés avec de l'eau chaude et pour séparer une seconde liqueur mère des cristaux,
(c) un réservoir d'eau pour fournir de l'eau chaude aux unités de filtration à membrane métallique frittée et aux seconds unités de filtration,
(d) une centrifugeuse pour centrifuger les cristaux filtrés par les secondes unités de filtration pour fournir une poudre cristalline d'acide naphthalène-2,6-dicarboxylic, et
(e) un séchoir pour sécher la poudre cristalline d'acide naphthalène-2,6-dicarboxylic.

7. Appareil selon la revendication 6, comprenant de plus un premier réservoir de liqueur mère en connexion avec les unités de filtration à membrane métallique frittée pour le stockage de la liqueur mère séparée par la séparation solide-liquide, un réservoir de liqueur mère de rinçage en connexion avec les secondes unités de filtration pour le stockage de la liqueur mère de rinçage provenant des secondes unités de filtration, et un réservoir disposé en amont de la centrifugeuse.

8. Appareil selon la revendication 6, dans lequel chacune des unités de filtration à membrane métallique frittée comprend deux orifices à travers desquels le solvant chaud à 200 à 250°C est introduit, un orifice à travers duquel le filtrat de liqueur mère est déchargé, une conduite d'équilibrage pour équilibrer les pressions du solvant introduite et de la suspension épaisse transférée, un orifice à travers duquel la suspension épaisse cristallisée est introduite, un orifice à travers duquel un mélange de la poudre cristalline séparée et du solvant est déchargé, un ouverture à travers duquel du gaz et de la vapeur sont évolués ou la pression se dégage, des manomètres pour contrôler la prestation et la pression du membrane, un thermomètre pour mesurer le température interne de l'unité, et des orifices de pressurisation pour régler la pression interne de l'unité.

9. Appareil selon la revendication 6, dans lequel chacune des unités de filtration à membrane métallique frittée comprend un filtre ayant une longueur de 50cm à 2 m, un diamètre de 10 à 50 cm et une épaisseur de 0.1 à 1 cm et un tube intérieur dont la longueur s'étend sensiblement jusqu'au fond du filtre et est 0.5 à 2 cm plus courte que celle du filtre.

10. Appareil selon la revendication 6, dans lequel les unités de filtration à membrane métallique frittée comprennent des filtres à membrane faits d'acier inoxydable, d'un alliage résistant à corrosion, ou d'un alliage résistant à la chaleur et ayant une taille de pores de 0.1 µm à 5 µm.
